# EUROPEAN PATENT SPECIFICATION

(11) **EP 3 216 390 B1**
(45) Date of publication and mention of the grant of the patent: **03.04.2024**
(21) Application number: 17167461.7
(22) Date of filing: 08.05.2008
(51) Int. Cl.: A61B 5/00, A61B 5/145

(54) **ANALYTE MONITORING SYSTEM**
ANALYTÜBERWACHUNGSSYSTEM
SYSTÈME DE SURVEILLANCE D'ANALYTE

(30) Priority: 08.05.2007 US 916776 P; 08.05.2007 US 916773 P; 08.05.2007 US 916761 P; 08.05.2007 US 916744 P; 08.05.2007 US 916723 P; 08.05.2007 US 916677 P
(43) Date of publication of application: 13.09.2017
(62) Divisional of application: 08755195.8
(73) Proprietor: Abbott Diabetes Care, Inc., Alameda, CA 94502 (US)
(72) Inventor: FENNELL, Martin, Concord, CA 94521 (US); HE, Lei, Moraga, CA 94556 (US); SLOAN, Mark Kent, Redwood City, CA 94065 (US); HAYTER, Gary Alan, Oakland, CA 94618 (US)
(74) Representative: Booth, Catherine Louise

(56) References cited:
- US-A- 5 830 129
- US-A1- 2002 013 538
- US-A1- 2003 004 403
- US-A1- 2004 106 859
- US-A1- 2007 038 044
- IEEE: "Guide for Health informatics-Point-of-care 3 medical device communication-Technical report- 4 Guidelines for the use of RF wireless technology", UNAPPROVED IEEE STANDARD,, no. P11073-00101(TM)/D02J, 1 February 2007 (2007-02-01), pages 1-111, XP007921782,
- Robert Istepanian ET AL: "M-Health: Emerging Mobile Health Systems" In: "M-Health: Emerging Mobile Health Systems", 16 November 2005 (2005-11-16), Springer, XP055406721, ISBN: 978-0-387-26558-2 pages ToC-Ch05,Ch08-Ch19, * page 72, paragraph 3 - page 77, paragraph 4 *

## Description

### BACKGROUND

Analyte, e.g., glucose monitoring systems including continuous and discrete monitoring systems generally include a small, lightweight battery powered and microprocessor controlled system which is configured to detect signals proportional to the corresponding measured glucose levels using an electrometer. RF signals may be used to transmit the collected data. One aspect of certain analyte monitoring systems include a transcutaneous or subcutaneous analyte sensor configuration which is, for example, at least partially positioned through the skin layer of a subject whose analyte level is to be monitored. The sensor may use a two or three-electrode (work, reference and counter electrodes) configuration driven by a controlled potential (potentiostat) analog circuit connected through a contact system.

An analyte sensor may be configured so that a portion thereof is placed under the skin of the patient so as to contact analyte of the patient, and another portion or segment of the analyte sensor may be in communication with the transmitter unit. The transmitter unit may be configured to transmit the analyte levels detected by the sensor over a wireless communication link such as an RF (radio frequency) communication link to a receiver/monitor unit. The receiver/monitor unit may perform data analysis, among other functions, on the received analyte levels to generate information pertaining to the monitored analyte levels.

Transmission of control or command data over wireless communication link is often constrained to occur within a substantially short time duration. In turn, the time constraint in data communication imposes limits on the type and size of data that may be transmitted during the transmission time period.

In view of the foregoing, it would be desirable to have a method and apparatus for optimizing the RF communication link between two or more communication devices, for example, in a medical communication system.

US-A-2003/0004403 describes a gateway platform for biological monitoring and delivery of therapeutic compounds. US-A-5830129 describes a process and apparatus for measuring blood flow through an organ or other biological tissue. US-A-2004/0106859 describes an analyte monitoring device and methods of use. US-A-2002/0013538 describes a method and apparatus for health signs monitoring. US-A-2007/0038044 describes an analyte sensor.

### SUMMARY

The present invention is defined by the appended claims. Devices and methods for analyte monitoring, e.g., glucose monitoring, are provided. Arrangements disclosed herein include transmitting information from a first location to a second, e.g., using a telemetry system such as RF telemetry. Systems disclosed herein include continuous analyte monitoring systems and discrete analyte monitoring system.

In one arrangement, a method including positioning a controller unit within a transmission range for close proximity communication, transmitting one or more predefined close proximity commands, and receiving a response packet in response to the transmitted one or more predefined close proximity commands, is disclosed, as well as devices and systems for the same.

In one arrangement, a method including detecting an electrical connection with an analyte sensor, and activating a data processing device to receive one or more analyte related signals from the analyte sensor, is disclosed, as well as devices and systems for the same.

In one arrangement, a method including receiving a command to initiate communication with an analyte sensor, retrieving an identification information, transmitting the retrieved identification information, and receiving a communication key associated with the transmitted identification information, is disclosed, as well as devices and systems for the same.

In one arrangement, a method including detecting a data transmission, incrementing a count associated with the detected data transmission, and storing the count, is disclosed, as well as devices and systems for the same.

In one arrangement, a method determining a communication status, receiving a communication related close proximity command, and modifying the communication status based on the received command, is disclosed, as well as devices and systems for the same.

In one arrangement, a method including periodically receiving data including a count information related to an analyte sensor associated with the received data, and comparing the count information in each data periodically received, and when it is determined that the count information of the periodically received data is different, generating one or more signals related to the status of the analyte sensor, is disclosed, as well as devices and systems for the same.

These and other objects, features and advantages of the present invention will become more fully apparent from the following detailed description of the embodiments, the appended claims and the accompanying drawings.

### BRIEF DESCRIPTION OF THE DRAWINGS

FIG. 1 illustrates a block diagram of a data monitoring and management system for practicing one or more embodiments of the present invention;
FIG. 2 is a block diagram of the transmitter unit of the data monitoring and management system shown in FIG. 1 in accordance with one embodiment of the present invention;
FIG. 3 is a block diagram of the receiver/monitor unit of the data monitoring and management system shown in FIG. 1 in accordance with one embodiment of the present invention;
FIG. 4 is a flowchart illustrating data packet procedure including rolling data for transmission in accordance with one embodiment of the present invention;
FIG. 5 is a flowchart illustrating data processing of the received data packet including the rolling data in accordance with one embodiment of the present invention;
FIG. 6 is a block diagram illustrating the sensor unit and the transmitter unit of the data monitoring and management system of FIG. 1 in accordance with one embodiment of the present invention;
FIG. 7 is a flowchart illustrating data communication using close proximity commands in the data monitoring and management system of FIG. 1 in accordance with one embodiment of the present invention;
FIG. 8 is a flowchart illustrating sensor insertion detection routine in the data monitoring and management system of FIG. 1 in accordance with one embodiment of the present invention;
FIG. 9 is a flowchart illustrating sensor removal detection routine in the data monitoring and management system of FIG. 1 in accordance with one embodiment of the present invention;
FIG. 10 is a flowchart illustrating the pairing or synchronization routine in the data monitoring and management system of FIG. 1 in accordance with one embodiment of the present invention;
FIG. 11 is a flowchart illustrating the pairing or synchronization routine in the data monitoring and management system of FIG. 1 in accordance with another embodiment of the present invention;
FIG. 12 is a flowchart illustrating the power supply determination in the data monitoring and management system of FIG. 1 in accordance with one embodiment of the present invention;
FIG. 13 is a flowchart illustrating close proximity command for RF communication control in the data monitoring and management system of FIG. 1 in accordance with one embodiment of the present invention; and
FIG. 14 is a flowchart illustrating analyte sensor identification routine in accordance with one embodiment of the present invention.

### DETAILED DESCRIPTION

As summarized above and as described in further detail below, in accordance with the various embodiments of the present invention, there is provided a method and system for positioning a controller unit within a transmission range for close proximity communication, transmitting one or more predefined close proximity commands, and receiving a response packet in response to the transmitted one or more predefined close proximity commands.

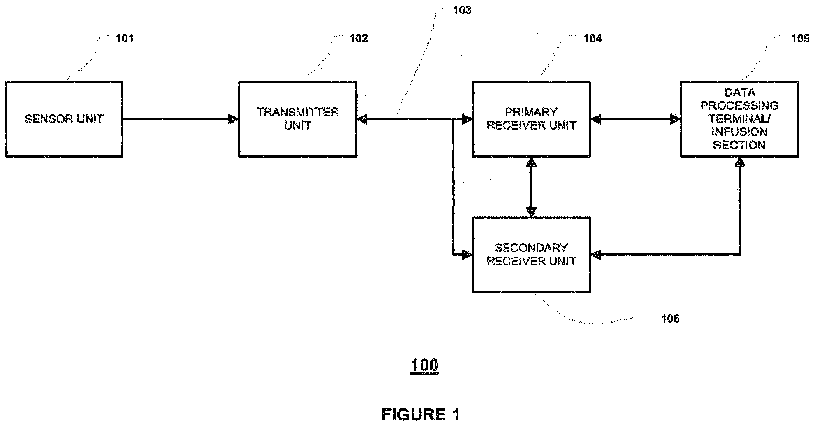

FIG. 1 illustrates a data monitoring and management system such as, for example, analyte (e.g., glucose) monitoring system 100 in accordance with one embodiment of the present disclosure. While the present invention is limited to glucose monitoring systems, it is to be understood that the analyte monitoring system may be configured to monitor a variety of analytes, e.g., lactate, and the like.

Analytes that may be monitored include, for example, acetyl choline, amylase, bilirubin, cholesterol, chorionic gonadotropin, creatine kinase (e.g., CK-MB), creatine, DNA, fructosamine, glucose, glutamine, growth hormones, hormones, ketones, lactate, peroxide, prostate-specific antigen, prothrombin, RNA, thyroid stimulating hormone, and troponin. The concentration of drugs, such as, for example, antibiotics (e.g., gentamicin, vancomycin, and the like), digitoxin, digoxin, drugs of abuse, theophylline, and warfarin, may also be monitored. More than one analyte may be monitored by a single system, e.g. a single analyte sensor.

The analyte monitoring system 100 includes a sensor unit 101, a transmitter unit 102 coupleable to the sensor unit 101, and a primary receiver unit 104 which is configured to communicate with the transmitter unit 102 via a bi-directional communication link 103. The primary receiver unit 104 may be further configured to transmit data to a data processing terminal 105 for evaluating the data received by the primary receiver unit 104. Moreover, the data processing terminal 105 in one embodiment may be configured to receive data directly from the transmitter unit 102 via a communication link which may optionally be configured for bi-directional communication. Accordingly, transmitter unit 102 and/or receiver unit 104 may include a transceiver.

Also shown in FIG. 1 is an optional secondary receiver unit 106 which is operatively coupled to the communication link and configured to receive data transmitted from the transmitter unit 102. Moreover, as shown in the Figure, the secondary receiver unit 106 is configured to communicate with the primary receiver unit 104 as well as the data processing terminal 105. Indeed, the secondary receiver unit 106 may be configured for bi-directional wireless communication with each or one of the primary receiver unit 104 and the data processing terminal 105. As discussed in further detail below, in one embodiment of the present invention, the secondary receiver unit 106 may be configured to include a limited number of functions and features as compared with the primary receiver unit 104. As such, the secondary receiver unit 106 may be configured substantially in a smaller compact housing or embodied in a device such as a wrist watch, pager, mobile phone, PDA, for example. Alternatively, the secondary receiver unit 106 may be configured with the same or substantially similar functionality as the primary receiver unit 104. The receiver unit may be configured to be used in conjunction with a docking cradle unit, for example for one or more of the following or other functions: placement by bedside, for re-charging, for data management, for night time monitoring, and/or bi-directional communication device.

In one aspect sensor unit 101 may include two or more sensors, each configured to communicate with transmitter unit 102. Furthermore, while only one, transmitter unit 102, communication link 103, and data processing terminal 105 are shown in the embodiment of the analyte monitoring system 100 illustrated in FIG. 1. However, it will be appreciated by one of ordinary skill in the art that the analyte monitoring system 100 may include one or more sensors, multiple transmitter units 102, communication links 103, and data processing terminals 105. Moreover, within the scope of the present invention, the analyte monitoring system 100 may be a continuous monitoring system, or semi-continuous, or a discrete monitoring system. In a multi-component environment, each device is configured to be uniquely identified by each of the other devices in the system so that communication conflict is readily resolved between the various components within the analyte monitoring system 100.

In one example, the sensor unit 101 is physically positioned in or on the body of a user whose analyte level is being monitored. The sensor unit 101 may be configured to continuously sample the analyte level of the user and convert the sampled analyte level into a corresponding data signal for transmission by the transmitter unit 102. In certain embodiments, the transmitter unit 102 may be physically coupled to the sensor unit 101 so that both devices are integrated in a single housing and positioned on the user's body. The transmitter unit 102 may perform data processing such as filtering and encoding on data signals and/or other functions, each of which corresponds to a sampled analyte level of the user, and in any event transmitter unit 102 transmits analyte information to the primary receiver unit 104 via the communication link 103.

In one embodiment, the analyte monitoring system 100 is configured as a one-way RF communication path from the transmitter unit 102 to the primary receiver unit 104. In such embodiment, the transmitter unit 102 transmits the sampled data signals received from the sensor unit 101 without acknowledgement from the primary receiver unit 104 that the transmitted sampled data signals have been received. For example, the transmitter unit 102 may be configured to transmit the encoded sampled data signals at a fixed rate (e.g., at one minute intervals) after the completion of the initial power on procedure. Likewise, the primary receiver unit 104 may be configured to detect such transmitted encoded sampled data signals at predetermined time intervals. Alternatively, the analyte monitoring system 100 may be configured with a bi-directional RF (or otherwise) communication between the transmitter unit 102 and the primary receiver unit 104.

Additionally, in one aspect, the primary receiver unit 104 may include two sections. The first section is an analog interface section that is configured to communicate with the transmitter unit 102 via the communication link 103. In one embodiment, the analog interface section may include an RF receiver and an antenna for receiving and amplifying the data signals from the transmitter unit 102, which are thereafter, demodulated with a local oscillator and filtered through a band-pass filter. The second section of the primary receiver unit 104 is a data processing section which is configured to process the data signals received from the transmitter unit 102 such as by performing data decoding, error detection and correction, data clock generation, and data bit recovery.

In operation, upon completing the power-on procedure, the primary receiver unit 104 is configured to detect the presence of the transmitter unit 102 within its range based on, for example, the strength of the detected data signals received from the transmitter unit 102 and/or a predetermined transmitter identification information. Upon successful synchronization with the corresponding transmitter unit 102, the primary receiver unit 104 is configured to begin receiving from the transmitter unit 102 data signals corresponding to the user's detected analyte level. More specifically, the primary receiver unit 104 in one embodiment is configured to perform synchronized time hopping with the corresponding synchronized transmitter unit 102 via the communication link 103 to obtain the user's detected analyte level.

Referring again to FIG. 1, the data processing terminal 105 may include a personal computer, a portable computer such as a laptop or a handheld device (e.g., personal digital assistants (PDAs)), and the like, each of which may be configured for data communication with the receiver via a wired or a wireless connection. Additionally, the data processing terminal 105 may further be connected to a data network (not shown) for storing, retrieving and updating data corresponding to the detected analyte level of the user.

Within the scope of the present invention, the data processing terminal 105 may include an infusion device such as an insulin infusion pump (external or implantable) or the like, which may be configured to administer insulin to patients, and which may be configured to communicate with the receiver unit 104 for receiving, among others, the measured analyte level. Alternatively, the receiver unit 104 may be configured to integrate or otherwise couple to an infusion device therein so that the receiver unit 104 is configured to administer insulin therapy to patients, for example, for administering and modifying basal profiles, as well as for determining appropriate boluses for administration based on, among others, the detected analyte levels received from the transmitter unit 102.

Additionally, the transmitter unit 102, the primary receiver unit 104 and the data processing terminal 105 may each be configured for bi-directional wireless communication such that each of the transmitter unit 102, the primary receiver unit 104 and the data processing terminal 105 may be configured to communicate (that is, transmit data to and receive data from) with each other via the wireless communication link 103. More specifically, the data processing terminal 105 may in one embodiment be configured to receive data directly from the transmitter unit 102 via the communication link 106, where the communication link 106, as described above, may be configured for bi-directional communication.

In this embodiment, the data processing terminal 105 which may include an insulin pump, may be configured to receive the analyte signals from the transmitter unit 102, and thus, incorporate the functions of the receiver 103 including data processing for managing the patient's insulin therapy and analyte monitoring. In one embodiment, the communication link 103 may include one or more of an RF communication protocol, an infrared communication protocol, a Bluetooth enabled communication protocol, an 802.1 1x wireless communication protocol, or an equivalent wireless communication protocol which would allow secure, wireless communication of several units (for example, per HIPPA requirements) while avoiding potential data collision and interference.

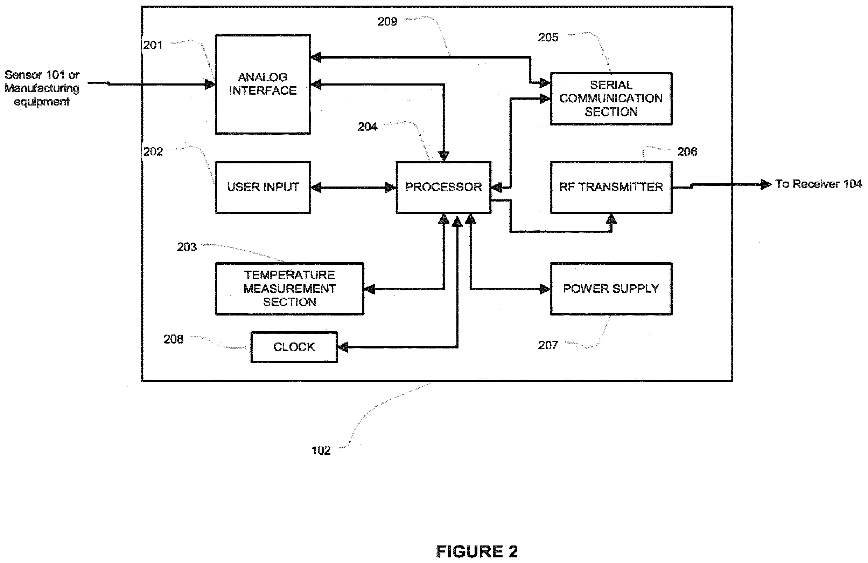

FIG. 2 is a block diagram of the transmitter of the data monitoring and detection system shown in FIG. 1 in accordance with one embodiment of the present invention. Referring to the Figure, the transmitter unit 102 in one embodiment includes an analog interface 201 configured to communicate with the sensor unit 101 (FIG. 1), a user input 202, and a temperature detection section 203, each of which is operatively coupled to a transmitter processor 204 such as a central processing unit (CPU). As can be seen from FIG. 2, there are provided four contacts, three of which are electrodes - work electrode (W) 210, guard contact (G) 211, reference electrode (R) 212, and counter electrode (C) 213, each operatively coupled to the analog interface 201 of the transmitter unit 102 for connection to the sensor unit 101 (FIG. 1). In one embodiment, each of the work electrode (W) 210, guard contact (G) 211, reference electrode (R) 212, and counter electrode (C) 213 may be made using a conductive material that is either printed or etched or ablated, for example, such as carbon which may be printed, or a metal such as a metal foil (e.g., gold) or the like, which may be etched or ablated or otherwise processed to provide one or more electrodes. Fewer or greater electrodes and/or contact may be provided in certain embodiments.

Further shown in FIG. 2 are a transmitter serial communication section 205 and an RF transmitter 206, each of which is also operatively coupled to the transmitter processor 204. Moreover, a power supply 207 such as a battery is also provided in the transmitter unit 102 to provide the necessary power for the transmitter unit 102. Additionally, as can be seen from the Figure, clock 208 is provided to, among others, supply real time information to the transmitter processor 204.

In one embodiment, a unidirectional input path is established from the sensor unit 101 (FIG. 1) and/or manufacturing and testing equipment to the analog interface 201 of the transmitter unit 102, while a unidirectional output is established from the output of the RF transmitter 206 of the transmitter unit 102 for transmission to the primary receiver unit 104. In this manner, a data path is shown in FIG. 2 between the aforementioned unidirectional input and output via a dedicated link 209 from the analog interface 201 to serial communication section 205, thereafter to the processor 204, and then to the RF transmitter 206. As such, in one embodiment, via the data path described above, the transmitter unit 102 is configured to transmit to the primary receiver unit 104 (FIG. 1), via the communication link 103 (FIG. 1), processed and encoded data signals received from the sensor unit 101 (FIG. 1). Additionally, the unidirectional communication data path between the analog interface 201 and the RF transmitter 206 discussed above allows for the configuration of the transmitter unit 102 for operation upon completion of the manufacturing process as well as for direct communication for diagnostic and testing purposes.

As discussed above, the transmitter processor 204 is configured to transmit control signals to the various sections of the transmitter unit 102 during the operation of the transmitter unit 102. In one embodiment, the transmitter processor 204 also includes a memory (not shown) for storing data such as the identification information for the transmitter unit 102, as well as the data signals received from the sensor unit 101. The stored information may be retrieved and processed for transmission to the primary receiver unit 104 under the control of the transmitter processor 204. Furthermore, the power supply 207 may include a commercially available battery, which may be a rechargeable battery.

In certain embodiments, the transmitter unit 102 is also configured such that the power supply section 207 is capable of providing power to the transmitter for a minimum of about three months of continuous operation, e.g., after having been stored for about eighteen months such as stored in a low-power (non-operating) mode. In one embodiment, this may be achieved by the transmitter processor 204 operating in low power modes in the non-operating state, for example, drawing no more than approximately 1 µA of current. Indeed, in one embodiment, a step during the manufacturing process of the transmitter unit 102 may place the transmitter unit 102 in the lower power, non-operating state (i.e., post-manufacture sleep mode). In this manner, the shelf life of the transmitter unit 102 may be significantly improved. Moreover, as shown in FIG. 2, while the power supply unit 207 is shown as coupled to the processor 204, and as such, the processor 204 is configured to provide control of the power supply unit 207, it should be noted that within the scope of the present invention, the power supply unit 207 is configured to provide the necessary power to each of the components of the transmitter unit 102 shown in FIG. 2.

Referring back to FIG. 2, the power supply section 207 of the transmitter unit 102 in one embodiment may include a rechargeable battery unit that may be recharged by a separate power supply recharging unit (for example, provided in the receiver unit 104) so that the transmitter unit 102 may be powered for a longer period of usage time. Moreover, in one embodiment, the transmitter unit 102 may be configured without a battery in the power supply section 207, in which case the transmitter unit 102 may be configured to receive power from an external power supply source (for example, a battery) as discussed in further detail below.

Referring yet again to FIG. 2, the temperature detection section 203 of the transmitter unit 102 is configured to monitor the temperature of the skin near the sensor insertion site. The temperature reading is used to adjust the analyte readings obtained from the analog interface 201. In certain embodiments, the RF transmitter 206 of the transmitter unit 102 may be configured for operation in the frequency band of approximately 315 MHz to approximately 322 MHz, for example, in the United States. In certain embodiments, the RF transmitter 206 of the transmitter unit 102 may be configured for operation in the frequency band of approximately 400MHz to approximately 470MHz. Further, in one embodiment, the RF transmitter 206 is configured to modulate the carrier frequency by performing Frequency Shift Keying and Manchester encoding. In one embodiment, the data transmission rate is about 19,200 symbols per second, with a minimum transmission range for communication with the primary receiver unit 104.

Referring yet again to FIG. 2, also shown is a leak detection circuit 214 coupled to the guard electrode (G) 211 and the processor 204 in the transmitter unit 102 of the data monitoring and management system 100. The leak detection circuit 214 in accordance with one embodiment of the present invention may be configured to detect leakage current in the sensor unit 101 to determine whether the measured sensor data are corrupt or whether the measured data from the sensor 101 is accurate. Exemplary analyte systems that may be employed are described in, for example, U.S. Patent Nos. 6,134,461, 6,175,752,6,121,611,6,560,471,6,746,582, and elsewhere.

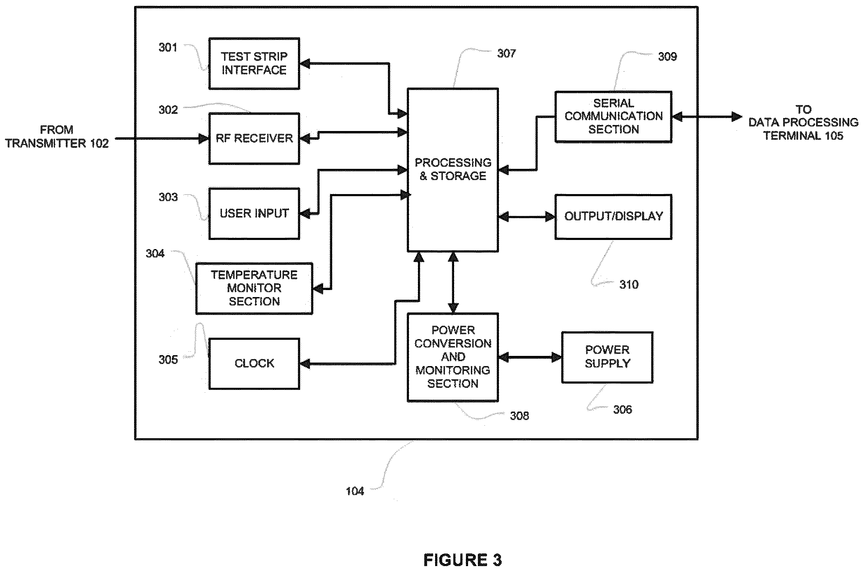

FIG. 3 is a block diagram of the receiver/monitor unit of the data monitoring and management system shown in FIG. 1 in accordance with one embodiment of the present invention. Referring to FIG. 3, the primary receiver unit 104 includes an analyte test strip, e.g., blood glucose test strip, interface 301, an RF receiver 302, an input 303, a temperature detection section 304, and a clock 305, each of which is operatively coupled to a receiver processor 307. As can be further seen from the Figure, the primary receiver unit 104 also includes a power supply 306 operatively coupled to a power conversion and monitoring section 308. Further, the power conversion and monitoring section 308 is also coupled to the receiver processor 307. Moreover, also shown are a receiver serial communication section 309, and an output 310, each operatively coupled to the receiver processor 307.

In one embodiment, the test strip interface 301 includes a glucose level testing portion to receive a manual insertion of a glucose test strip, and thereby determine and display the glucose level of the test strip on the output 310 of the primary receiver unit 104. This manual testing of glucose may be used to calibrate the sensor unit 101 or otherwise. The RF receiver 302 is configured to communicate, via the communication link 103 (FIG. 1) with the RF transmitter 206 of the transmitter unit 102, to receive encoded data signals from the transmitter unit 102 for, among others, signal mixing, demodulation, and other data processing. The input 303 of the primary receiver unit 104 is configured to allow the user to enter information into the primary receiver unit 104 as needed. In one aspect, the input 303 may include one or more keys of a keypad, a touch-sensitive screen, or a voice-activated input command unit. The temperature detection section 304 is configured to provide temperature information of the primary receiver unit 104 to the receiver processor 307, while the clock 305 provides, among others, real time information to the receiver processor 307.

Each of the various components of the primary receiver unit 104 shown in FIG. 3 is powered by the power supply 306 which, in one embodiment, includes a battery. Furthermore, the power conversion and monitoring section 308 is configured to monitor the power usage by the various components in the primary receiver unit 104 for effective power management and to alert the user, for example, in the event of power usage which renders the primary receiver unit 104 in sub-optimal operating conditions. An example of such sub-optimal operating condition may include, for example, operating the vibration output mode (as discussed below) for a period of time thus substantially draining the power supply 306 while the processor 307 (thus, the primary receiver unit 104) is turned on. Moreover, the power conversion and monitoring section 308 may additionally be configured to include a reverse polarity protection circuit such as a field effect transistor (FET) configured as a battery activated switch.

The serial communication section 309 in the primary receiver unit 104 is configured to provide a bi-directional communication path from the testing and/or manufacturing equipment for, among others, initialization, testing, and configuration of the primary receiver unit 104. Serial communication section 104 can also be used to upload data to a computer, such as time-stamped blood glucose data. The communication link with an external device (not shown) can be made, for example, by cable, infrared (IR) or RF link. The output 310 of the primary receiver unit 104 is configured to provide, among others, a graphical user interface (GUI) such as a liquid crystal display (LCD) for displaying information. Additionally, the output 310 may also include an integrated speaker for outputting audible signals as well as to provide vibration output as commonly found in handheld electronic devices, such as mobile telephones presently available. In a further embodiment, the primary receiver unit 104 also includes an electro-luminescent lamp configured to provide backlighting to the output 310 for output visual display in dark ambient surroundings.

Referring back to FIG. 3, the primary receiver unit 104 in one embodiment may also include a storage section such as a programmable, non-volatile memory device as part of the processor 307, or provided separately in the primary receiver unit 104, operatively coupled to the processor 307. The processor 307 may be configured to synchronize with a transmitter, e.g., using Manchester decoding or the like, as well as error detection and correction upon the encoded data signals received from the transmitter unit 102 via the communication link 103.

Additional description of the RF communication between the transmitter 102 and the primary receiver 104 (or with the secondary receiver 106) that may be employed in embodiments is disclosed in application no. 11/060,365 (publication no. US-2005/0182306) filed February 16,2005 entitled "Method and System for Providing Data Communication in Continuous Glucose Monitoring and Management System".

Referring to the Figures, the transmitter 102 (FIG. 1) is configured to generate data packets for, e.g. periodic, transmission to one ore more of the receiver units 104, 106, where each data packet includes two categories of data - urgent data and non-urgent data. Urgent data such as for example glucose data from the sensor and/or temperature data associated with the sensor is packed in each data packet in addition to non-urgent data, where the non-urgent data may be rolled or varied with each data packet transmission.

That is, the non-urgent data is transmitted at a timed interval so as to maintain the integrity of the analyte monitoring system without being transmitted over the RF communication link with each data transmission packet from the transmitter 102. In this manner, the non-urgent data, for example that are not time sensitive, may be periodically transmitted (and not with each data packet transmission), having been broken up into predetermined number of segments and sent or transmitted over multiple packets, while the urgent data is transmitted substantially in its entirety with each data transmission.

Referring again to the Figures, upon receiving the data packets from the transmitter 102, the one or more receiver units 104, 106 are configured to parse the received the data packet to separate the urgent data from the non-urgent data, and also, may be configured to store the urgent data and the non-urgent data, e.g., in a hierarchical manner. In accordance with the particular configuration of the data packet or the data transmission protocol, more or less data may be transmitted as part of the urgent data, or the non-urgent rolling data. That is, within the scope of the present disclosure, the specific data packet implementation such as the number of bits per packet, and the like, may vary based on, among others, the communication protocol, data transmission time window, and so on.

In an exemplary embodiment, different types of data packets may be identified accordingly. For example, identification in certain exemplary embodiments may include - (1) single sensor, one minute of data, (2) two or multiple sensors, (3) dual sensor, alternate one minute data, and (4) response packet. For single sensor one minute data packet, in one embodiment, the transmitter 102 may be configured to generate the data packet in the manner, or similar to the manner, shown in Table 1 below.

**Table 1. Single sensor, one minute of data**

| **Number of Bits** | **Data Field** |
|---|---|
| 8 | Transmit Time |
| 14 | Sensor1 Current Data |
| 14 | Sensor1 Historic Data |
| 8 | Transmit Status |
| 12 | AUX Counter |
| 12 | AUX Thermistor 1 |
| 12 | AUX Thermistor 2 |
| 8 | Rolling-Data-1 |

As shown in Table 1 above, the transmitter data packet in one embodiment may include 8 bits of transmit time data, 14 bits of current sensor data, 14 bits of preceding sensor data, 8 bits of transmitter status data, 12 bits of auxiliary counter data, 12 bits of auxiliary thermistor 1 data, 12 bits of auxiliary thermistor 1 data and 8 bits of rolling data. In one embodiment of the present invention, the data packet generated by the transmitter for transmission over the RF communication link may include all or some of the data shown above in Table 1.

Referring back, the 14 bits of the current sensor data provides the real time or current sensor data associated with the detected analyte level, while the 14 bits of the sensor historic or preceding sensor data includes the sensor data associated with the detected analyte level one minute ago. In this manner, in the case where the receiver unit 104, 106 drops or fails to successfully receive the data packet from the transmitter 102 in the minute by minute transmission, the receiver unit 104, 106 may be able to capture the sensor data of a prior minute transmission from a subsequent minute transmission.

Referring again to Table 1, the Auxiliary data in one embodiment may include one or more of the patient's skin temperature data, a temperature gradient data, reference data, and counter electrode voltage. The transmitter status field may include status data that is configured to indicate corrupt data for the current transmission (for example, if shown as BAD status (as opposed to GOOD status which indicates that the data in the current transmission is not corrupt)). Furthermore, the rolling data field is configured to include the non-urgent data, and in one embodiment, may be associated with the time-hop sequence number. In addition, the Transmitter Time field in one embodiment includes a protocol value that is configured to start at zero and is incremented by one with each data packet. In one aspect, the transmitter time data may be used to synchronize the data transmission window with the receiver unit 104, 106, and also, provide an index for the Rolling data field.

In a further embodiment, the transmitter data packet may be configured to provide or transmit analyte sensor data from two or more independent analyte sensors. The sensors may relate to the same or different analyte or property. In such a case, the data packet from the transmitter 102 may be configured to include 14 bits of the current sensor data from both sensors in the embodiment in which 2 sensors are employed. In this case, the data packet does not include the immediately preceding sensor data in the current data packet transmission. Instead, a second analyte sensor data is transmitted with a first analyte sensor data.

**Table 2. Dual sensor data**

| **Number of Bits** | **Data Field** |
|---|---|
| 8 | Transmit Time |
| 14 | Sensor1 Current Data |
| 14 | Sensor2 Current Data |
| 8 | Transmit Status |
| 12 | AUX Counter |
| 12 | AUX Thermistor 1 |
| 12 | AUX Thermistor 2 |
| 8 | Rolling-Data-1 |

In a further embodiment, the transmitter data packet may be alternated with each transmission between two analyte sensors, for example, alternating between the data packet shown in Table 3 and Table 4 below.

**Table 3. Sensor Data Packet Alternate 1**

| **Number of Bits** | **Data Field** |
|---|---|
| 8 | Transmitter Time |
| 14 | Sensor1 Current Data |
| 14 | Sensor1 Historic Data |
| 8 | Transmit Status |
| 12 | AUX Counter |
| 12 | AUX Thermistor 1 |
| 12 | AUX Thermistor 2 |
| 8 | Rolling-Data-1 |

**Table 4. Sensor Data Packet Alternate 2**

| **Number of Bits** | **Data Field** |
|---|---|
| 8 | Transmitter Time |
| 14 | Sensor1 Current Data |
| 14 | Sensor2 Current Data |
| 8 | Transmit Status |
| 12 | AUX Counter |
| 12 | AUX Thermistor 1 |
| 12 | AUX Thermistor 2 |
| 8 | Rolling-Data-1 |

As shown above in reference to Tables 3 and 4, the minute by minute data packet transmission from the transmitter 102 (FIG. 1) in one embodiment may alternate between the data packet shown in Table 3 and the data packet shown in Table 4. More specifically, the transmitter 102 may be configured in one embodiment transmit the current sensor data of the first sensor and the preceding sensor data of the first sensor (Table 3), as well as the rolling data, and further, at the subsequent transmission, the transmitter 102 may be configured to transmit the current sensor data of the first and the second sensor in addition to the rolling data.

In one embodiment, the rolling data transmitted with each data packet may include a sequence of various predetermined types of data that are considered not-urgent or not time sensitive. That is, in one embodiment, the following list of data shown in Table 5 may be sequentially included in the 8 bits of transmitter data packet, and not transmitted with each data packet transmission of the transmitter (for example, with each 60 second data transmission from the transmitter 102).

**Table 5. Rolling Data**

| **Time Slot** | **Bits** | **Rolling-Data** |
|---|---|---|
| 0 | 8 | Mode |
| 1 | 8 | Glucose1 Slope |
| 2 | 8 | Glucose2 Slope |
| 3 | 8 | Ref-R |
| 4 | 8 | Hobbs Counter, Ref-R |
| 5 | 8 | Hobbs Counter |
| 6 | 8 | Hobbs Counter |
| 7 | 8 | Sensor Count |

As can be seen from Table 5 above, in one embodiment, a sequence of rolling data are appended or added to the transmitter data packet with each data transmission time slot. In one embodiment, there may be 256 time slots for data transmission by the transmitter 102 (FIG. 1), and where, each time slot is separately by approximately 60 second interval. For example, referring to the Table 5 above, the data packet in transmission time slot 0 (zero) may include operational mode data (Mode) as the rolling data that is appended to the transmitted data packet. At the subsequent data transmission time slot (for example, approximately 60 seconds after the initial time slot (0), the transmitted data packet may include the analyte sensor 1 calibration factor information (Glucose 1 slope) as the rolling data. In this manner, with each data transmission, the rolling data may be updated over the 256 time slot cycle.

Referring again to Table 5, each rolling data field is described in further detail for various embodiments. For example, the Mode data may include information related to the different operating modes such as, but not limited to, the data packet type, the type of battery used, diagnostic routines, single sensor or multiple sensor input, type of data transmission (rf communication link or other data link such as serial connection). Further, the Glucose1-slope data may include an 8-bit scaling factor or calibration data for first sensor (scaling factor for sensor 1 data) , while Glucose2-slope data may include an 8-bit scaling factor or calibration data for the second analyte sensor (in the embodiment including more than one analyte sensors).

In addition, the Ref-R data may include 12 bits of on-board reference resistor used to calibrate our temperature measurement in the thermister circuit (where 8 bits are transmitted in time slot 3, and the remaining 4 bits are transmitted in time slot 4), and the 20-bit Hobbs counter data may be separately transmitted in three time slots (for example, in time slot 4, time slot 5 and time slot 6) to add up to 20 bits. In one embodiment, the Hobbs counter may be configured to count each occurrence of the data transmission (for example, a packet transmission at approximately 60 second intervals) and may be incremented by a count of one (1).

In one aspect, the Hobbs counter is stored in a nonvolatile memory of the transmitter unit 102 (FIG. 1) and may be used to ascertain the power supply status information such as, for example, the estimated battery life remaining in the transmitter unit 102. That is, with each sensor replacement, the Hobbs counter is not reset, but rather, continues the count with each replacement of the sensor unit 101 to establish contact with the transmitter unit 102 such that, over an extended usage time period of the transmitter unit 102, it may be possible to determine, based on the Hobbs count information, the amount of consumed battery life in the transmitter unit 102, and also, an estimated remaining life of the battery in the transmitter unit 102.

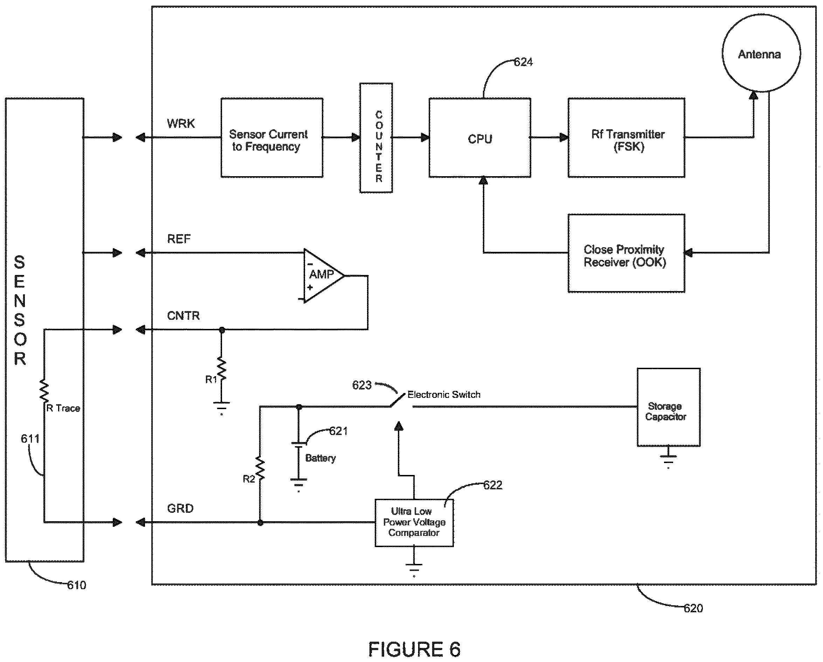

That is, in one embodiment, the 20 bit Hobbs counter is incremented by one each time the transmitter unit 102 transmits a data packet (for example, approximately each 60 seconds), and based on the count information in the Hobbs counter, in one aspect, the battery life of the transmitter unit 102 may be estimated. In this manner, in configurations of the transmitter unit 620 (see FIG. 6) where the power supply is not a replaceable component but rather, embedded within the housing the transmitter unit 620, it is possible to estimate the remaining life of the embedded battery within the transmitter unit 620. Moreover, the Hobbs counter is configured to remain persistent in the memory device of the transmitter unit 620 such that, even when the transmitter unit power is turned off or powered down (for example, during the periodic sensor unit replacement, RF transmission turned off period and the like), the Hobbs counter information is retained.

Referring to Table 5 above, the transmitted rolling data may also include 8 bits of sensor count information (for example, transmitted in time slot 7). The 8 bit sensor counter is incremented by one each time a new sensor unit is connected to the transmitter unit. The ASIC configuration of the transmitter unit (or a microprocessor based transmitter configuration or with discrete components) may be configured to store in a nonvolatile memory unit the sensor count information and transmit it to the primary receiver unit 104 (for example). In turn, the primary receiver unit 104 (and/or the secondary receiver unit 106) may be configured to determine whether it is receiving data from the transmitter unit that is associated with the same sensor unit (based on the sensor count information), or from a new or replaced sensor unit (which will have a sensor count incremented by one from the prior sensor count). In this manner, in one aspect, the receiver unit (primary or secondary) may be configured to prevent reuse of the same sensor unit by the user based on verifying the sensor count information associated with the data transmission received from the transmitter unit 102. In addition, in a further aspect, user notification may be associated with one or more of these parameters. Further, the receiver unit (primary or secondary) may be configured to detect when a new sensor has been inserted, and thus prevent erroneous application of one or more calibration parameters determined in conjunction with a prior sensor, that may potentially result in false or inaccurate analyte level determination based on the sensor data.

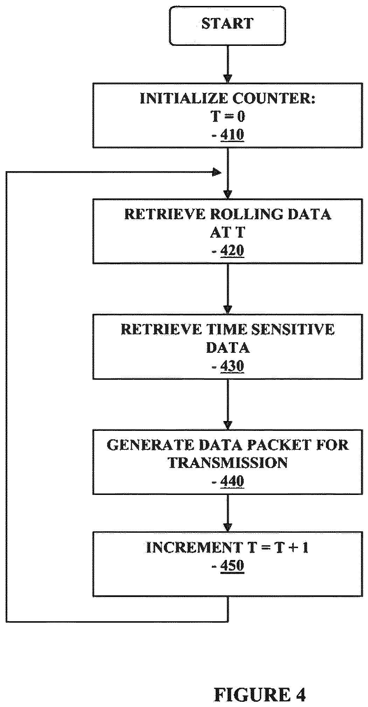

FIG. 4 is a flowchart illustrating a data packet procedure including rolling data for transmission in accordance with one embodiment of the present invention. Referring to FIG. 4, in one embodiment, a counter is initialized (for example, to T = 0) (410). Thereafter the associated rolling data is retrieved from memory device, for example (420), and also, the time sensitive or urgent data is retrieved (430). In one embodiment, the retrieval of the rolling data (420) and the retrieval of the time sensitive data (430) may be retrieved at substantially the same time.

Referring back to FIG. 4, with the rolling data and the time sensitive data, for example, the data packet for transmission is generated (440), an upon transmission, the counter is incremented by one and the routine returns to retrieval of the rolling data (420). In this manner, in one embodiment, the urgent time sensitive data as well as the non-urgent data may be incorporated in the same data packet and transmitted by the transmitter 102 (FIG. 1) to a remote device such as one or more of the receivers 104, 106. Furthermore, as discussed above, the rolling data may be updated at a predetermined time interval which is longer than the time interval for each data packet transmission from the transmitter 102 (FIG. 1).

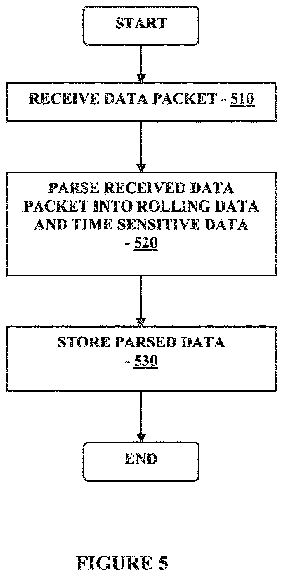

FIG. 5 is a flowchart illustrating data processing of the received data packet including the rolling data in accordance with embodiments of the present invention. Referring to FIG. 5, when the data packet is received (510) (for example, by one or more of the receivers 104, 106, in one embodiment) the received data packet is parsed so that the urgent data is separated from the non-urgent data (stored in, for example, the rolling data field in the data packet). Thereafter the parsed data may be suitably stored in an appropriate memory or storage device (530).

In the manner described above, in accordance with embodiments of the present invention, there is provided method and apparatus for separating non-urgent type data (for example, data associated with calibration) from urgent type data (for example, monitored analyte related data) to be transmitted over the communication link to minimize the potential burden or constraint on the available transmission time. More specifically, according to the present invention, non-urgent data is separated from data that is required by the communication system to be transmitted immediately, and transmitted over the communication link together while maintaining a minimum transmission time window. According to the present invention, the non-urgent data is parsed in to a number of data segments, and transmitted over multiple data packets. The time sensitive immediate data (for example, the analyte sensor data, temperature data etc), is transmitted over the communication link substantially in its entirety with each data packet or transmission.

FIG. 6 is a block diagram illustrating the sensor unit and the transmitter unit of the data monitoring and management system of FIG. 1 in accordance with one example. Referring to FIG. 6, in one aspect, a transmitter unit 620 is provided in a substantially water tight and sealed housing. The transmitter unit 620 includes respective contacts (wrk, Ref, Cntr, and gnd) for respectively establishing electrical contact with one or more of the working electrode, the reference electrode, the counter electrode and the ground terminal (or guard trace) of the sensor unit 610. Also shown in FIG. 6 is a conductivity bar/trace 611 provided on the sensor unit 610. For example, in one embodiment, the conductivity bar/trace 611 may comprise a carbon trace on a substrate layer of the sensor unit 610. In this manner, in one embodiment, when the sensor unit 610 is coupled to the transmitter unit 610, electrical contact is established, for example, via the conductivity bar/trace 611 between the contact pads or points of the transmitter unit 620 (for example, at the counter electrode contact (cntr) and the ground terminal contact (gnd) such that the transmitter unit 620 may be powered for data communication.

That is, during manufacturing of the transmitter unit 620, in one aspect, the transmitter unit 620 is configured to include a power supply such as battery 621. Further, during the initial non-use period (e.g., post manufacturing sleep mode), the transmitter unit 620 is configured such that it is not used and thus drained by the components of the transmitter unit 620. During the sleep mode, and prior to establishing electrical contact with the sensor unit 610 via the conductivity bar/trace 611, the transmitter unit 620 is provided with a low power signal from, for example, a low power voltage comparator 622, via an electronic switch 623 to maintain the low power state of, for example, the transmitter unit 620 components. Thereafter, upon connection with the sensor unit 610, and establishing electrical contact via the conductivity bar/trace 611, the embedded power supply 621 of the transmitter unit 620 is activated or powered up so that some of all of the components of the transmitter unit 620 are configured to receive the necessary power signals for operations related to, for example, data communication, processing and/or storage.

In one aspect, since the transmitter unit 620 is configured to a sealed housing without a separate replaceable battery compartment, in this manner, the power supply of the battery 621 is preserved during the post manufacturing sleep mode prior to use.

In a further aspect, the transmitter unit 620 may be disposed or positioned on a separate on-body mounting unit that may include, for example, an adhesive layer (on its bottom surface) to firmly retain the mounting unit on the skin of the user, and which is configured to receive or firmly position the transmitter unit 620 on the mounting unit during use. In one aspect, the mounting unit may be configured to at least partially retain the position of the sensor unit 610 in a transcutaneous manner so that at least a portion of the sensor unit is in fluid contact with the analyte of the user. Example embodiments of the mounting or base unit and its cooperation or coupling with the transmitter unit are provided, for example, in US Patent No. 6,175,752.

In such a configuration, the power supply for the transmitter unit 620 may be provided within the housing of the mounting unit such that, the transmitter unit 620 may be configured to be powered on or activated upon placement of the transmitter unit 620 on the mounting unit and in electrical contact with the sensor unit 610. For example, the sensor unit 610 may be provided pre-configured or integrated with the mounting unit and the insertion device such that, the user may position the sensor unit 610 on the skin layer of the user using the insertion device coupled to the mounting unit. Thereafter, upon transcutaneous positioning of the sensor unit 610, the insertion device may be discarded or removed from the mounting unit, leaving behind the transcutaneously positioned sensor unit 610 and the mounting unit on the skin surface of the user.

Thereafter, when the transmitter unit 620 is positioned on, over or within the mounting unit, the battery or power supply provided within the mounting unit is configured to electrically couple to the transmitter unit 620 and/or the sensor unit 610. Given that the sensor unit 610 and the mounting unit are provided as replaceable components for replacement every 3,5, 7 days or other predetermined time periods, the user is conveniently not burdened with verifying the status of the power supply providing power to the transmitter unit 620 during use. That is, with the power supply or battery replaced with each replacement of the sensor unit 610, a new power supply or battery will be provided with the new mounting unit for use with the transmitter unit 620.

Referring to FIG. 6 again, in one aspect, when the sensor unit 610 is removed from the transmitter unit 620 (or vice versa), the electrical contact is broken and the conductivity bar/trace 611 returns to an open circuit. In this case, the transmitter unit 620 may be configured, to detect such condition and generate a last gasp transmission sent to the primary receiver unit 104 (and/or the secondary receiver unit 106) indicating that the sensor unit 610 is disconnected from the transmitter unit 620, and that the transmitter unit 620 is entering a powered down (or low power off) state.

And the transmitter unit 620 is powered down into the sleep mode since the connection to the power supply (that is embedded within the transmitter unit 620 housing) is broken.

In this manner, in one aspect, the processor 624 of the transmitter unit 620 may be configured to generate the appropriate one or more data or signals associated with the detection of sensor unit 610 disconnection for transmission to the receiver unit 104 (FIG. 1), and also, to initiate the power down procedure of the transmitter unit 620. In one aspect, the components of the transmitter unit 620 may be configured to include application specific integrated circuit (ASIC) design with one or more state machines and one or more nonvolatile and/or volatile memory units such as, for example, EEPROMs and the like.

Referring again to FIGS. 1 and 6, in one embodiment, the communication between the transmitter unit 620 (or 102 of FIG. 1) and the primary receiver unit 104 (and/or the secondary receiver unit 106) may be based on close proximity communication where bi-directional (or uni-directional) wireless communication is established when the devices are physically located in close proximity to each other. That is, in one embodiment, the transmitter unit 620 may be configured to receive very short range commands from the primary receiver unit 104 (FIG. 1) and perform one or more specific operations based on the received commands from the receiver unit 104).

In one embodiment, to maintain secure communication between the transmitter unit and the data receiver unit, the transmitter unit ASIC may be configured to generate a unique close proximity key at power on or initialization. In one aspect, the 4 or 8 bit key may be generated based on, for example, the transmitter unit identification information, and which may be used to prevent undesirable or unintended communication. In a further aspect, the close proximity key may be generated by the receiver unit based on, for example, the transmitter identification information received by the transmitter unit during the initial synchronization or pairing procedure of the transmitter and the receiver units.

Referring again to FIGS. 1 and 6, in one embodiment, the transmitter unit ASIC configuration may include a 32KHz oscillator and a counter which may be configured to drive the state machine in the transmitter unit ASIC. The transmitter ASIC configuration may include a plurality of close proximity communication commands including, for example, new sensor initiation, pairing with the receiver unit, and RF communication control, among others. For example, when a new sensor unit is positioned and coupled to the transmitter unit so that the transmitter unit is powered on, the transmitter unit is configured to detect or receive a command from the receiver unit positioned in close proximity to the transmitter unit. For example, the receiver unit may be positioned within a couple of centimeters (inches) from the on-body position of the transmitter unit, and when the user activates or initiates a command associated with the new sensor initiation from the receiver unit, the transmitter unit is configured to receive the command from the receiver and, in its response data packet, transmit, among others, its identification information back to the receiver unit.

In one embodiment, the initial sensor unit initiation command does not require the use of the close proximity key. However, other predefined or preconfigured close-proximity commands may be configured to require the use of the 8 bit key (or a key of a different number of bits). For example, in one embodiment, the receiver unit may be configured to transmit a RF on/off command to turn on/off the RF communication module or unit in the transmitter unit 102. Such RF on/off command in one embodiment includes the close proximity key as part of the transmitted command for reception by the transmitter unit.

During the period that the RF communication module or unit is turned off based on the received close proximity command, the transmitter unit does not transmit any data, including any glucose related data. In one embodiment, the glucose related data from the sensor unit which are not transmitted by the transmitter unit during the time period when the RF communication module or unit of the transmitter unit is turned off may be stored in a memory or storage unit of the transmitter unit for subsequent transmission to the receiver unit when the transmitter unit RF communication module or unit is turned back on based on the RF-on command from the receiver unit. In this manner, in one embodiment, the transmitter unit may be powered down (temporarily, for example, during air travel) without removing the transmitter unit from the on-body position.

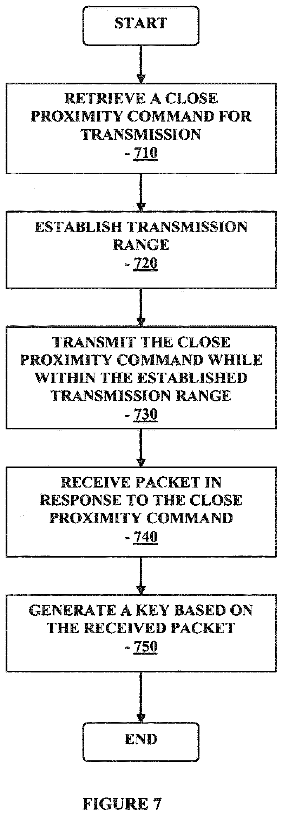

FIG. 7 is a flowchart illustrating data communication using close proximity commands in the data monitoring and management system of FIG. 1 in accordance with one embodiment of the present invention. Referring to FIG. 7, the primary receiver unit 104 (FIG. 1) in one aspect may be configured to retrieve or generate a close proximity command (710) for transmission to the transmitter unit 102. To establish the transmission range (720), the primary receiver unit 104 may be positioned physically close to (that is, within a predetermined distance from) the transmitter unit 102. For example, the transmission range for the close proximity communication may be established at approximately 30 cm (one foot) distance or less between the transmitter unit 102 and the primary receiver unit 104. When the transmitter unit 102 and the primary receiver unit 104 are within the transmission range, the close proximity command, upon initiation from the receiver unit 104 may be transmitted to the transmitter unit 102 (730).

Referring back to FIG. 7, in response to the transmitted close proximity command, a response data packet or other responsive communication may be received (740). In one aspect, the response data packet or other responsive communication may include identification information of the transmitter unit 102 transmitting the response data packer or other response communication to the receiver unit 104. In one aspect, the receiver unit 104 may be configured to generate a key (for example, an 8 bit key or a key of a predetermined length) based on the transmitter identification information (750), and which may be used in subsequent close proximity communication between the transmitter unit 102 and the receiver unit 104.

In one aspect, the data communication including the generated key may allow the recipient of the data communication to recognize the sender of the data communication and confirm that the sender of the data communication is the intended data sending device, and thus, including data which is desired or anticipated by the recipient of the data communication. In this manner, in one embodiment, one or more close proximity commands may be configured to include the generated key as part of the transmitted data packet. Moreover, the generated key may be based on the transmitter ID or other suitable unique information so that the receiver unit 104 may use such information for purposes of generating the unique key for the bi-directional communication between the devices.

While the description above includes generating the key based on the transmitter unit 102 identification information, within the scope of the present disclosure, the key may be generated based on one or more other information associated with the transmitter unit 102, and/or the receiver unit combination. In a further embodiment, the key may be encrypted and stored in a memory unit or storage device in the transmitter unit 102 for transmission to the receiver unit 104.

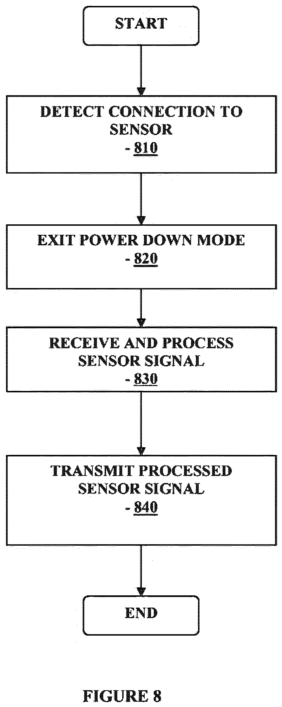

FIG. 8 is a flowchart illustrating sensor insertion detection routine in the data monitoring and management system of FIG. 1 in accordance with one embodiment of the present invention. Referring to FIG. 8, connection to an analyte sensor is detected (810, based on for example, a power up procedure where the sensor conduction trace 611 (FIG. 6) is configured to establish electrical contact with a predetermined one or more contact points on the transmitter unit 102. That is, when the sensor unit 101 (for example, the electrodes of the sensor) is correspondingly connected to the contact points on the transmitter unit 102, the transmitter unit 102 is configured to close the circuit connecting its power supply (for example, the battery 621 (FIG. 6) to the components of the transmitter unit 102 and thereby exiting the power down or low power state into active or power up state.

In this manner, as discussed above, in one aspect, the transmitter unit 102 may be configured to include a power supply such as a battery 621 integrally provided within the sealed housing of the transmitter unit 102. When the transmitter unit 102 is connected or coupled to the respective electrodes of the analyte sensor that is positioned in a transcutaneous manner under the skin layer of the patient, the transmitter unit 102 is configured to wake up from its low power or sleep state (820), and power up the various components of the transmitter unit 102. In the active state, the transmitter unit 102 may be further configured to receive and process sensor signals received from the analyte sensor (FIG. 1) (830), and thereafter, transmit the processed sensor signals (840) to, for example, the receiver unit 104 (FIG. 1).

Accordingly, in one aspect, the sensor unit 610 (FIG. 6) may be provided with a conduction trace 611 which may be used to wake up or exit the transmitter unit from its post manufacturing sleep mode into an active state, by for example, establishing a closed circuit with the power supply provided within the transmitter unit 102.

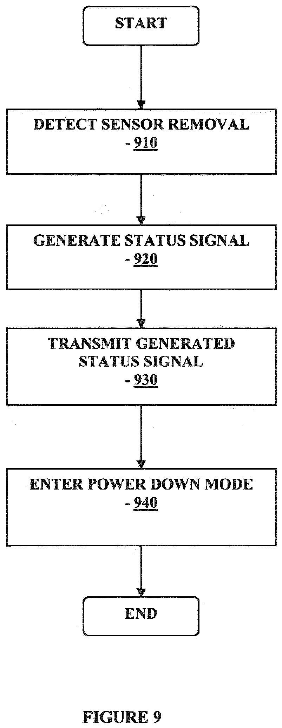

FIG. 9 is a flowchart illustrating sensor removal detection routine in the data monitoring and management system of FIG. 1 in accordance with one embodiment of the present invention. Referring to FIG. 9, when the sensor removal is detected (910) for example, based on detaching or removing the transmitter unit 102 that was in contact with the sensor unit 101, one or more status signal is generated (920), that includes, for example, an indication that the sensor removal state has been detected, and/or an indication that the transmitter unit 102 will enter a sleep mode or a powered down status. Thereafter, the generated status signal in one aspect is transmitted, for example, to the receiver unit 104 (930), and the transmitter unit 102 is configured to enter the power down mode or low power sleep mode (940).

In this manner, in one aspect, when the transmitter unit 102 is disconnected with an active sensor unit 101, the transmitter unit 102 is configured to notify the receiver unit 104 that the sensor unit 101 has been disconnected or otherwise, signals from the sensor unit 101 is no longer received by the transmitter unit 102. After transmitting the one or more signals to notify the receiver unit 104, the transmitter unit 102 in one embodiment is configured to enter sleep mode or low power state during which no data related to the monitored analyte level is transmitted to the receiver unit 104.

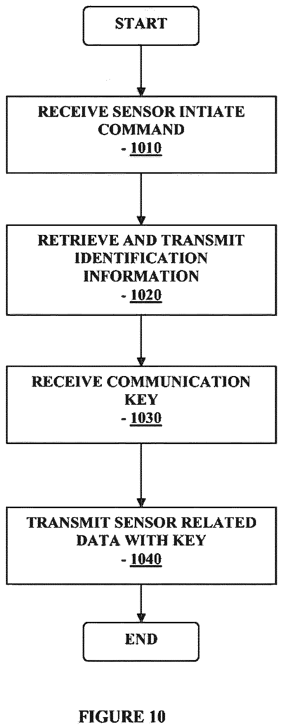

FIG. 10 is a flowchart illustrating the pairing or synchronization routine in the data monitoring and management system of FIG. 1 in accordance with one embodiment of the present invention. Referring to FIG. 10, in one embodiment, the transmitter unit 102 may be configured to receive a sensor initiate close proximity command (1010) from the receiver unit 104 positioned within the close transmission range. Based on the received sensor initiate command, the transmitter unit identification information may be retrieved (for example, from a nonvolatile memory) and transmitted (1020) to the receiver unit 104 or the sender of the sensor initiate command.

Referring back to FIG. 10, a communication key (1030) optionally encrypted is received in one embodiment, and thereafter, sensor related data is transmitted with the communication key on a periodic basis such as, every 60 seconds, five minutes, or any suitable predetermined time intervals.

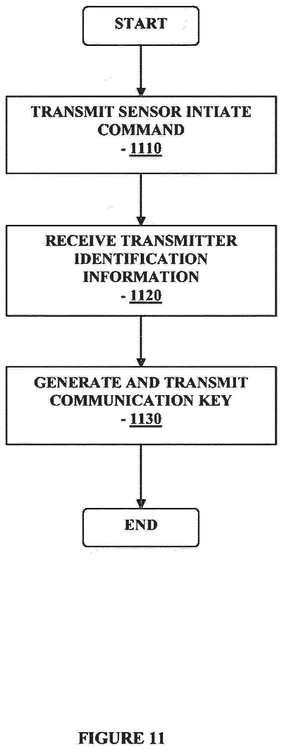

Referring now to FIG. 11, a flowchart illustrating the pairing or synchronization routine in the data monitoring and management system of FIG. 1 in accordance with another embodiment of the present invention is shown. That is, in one aspect, FIG. 11 illustrates the pairing or synchronization routine from the receiver unit 104. Referring back to FIG. 11, the sensor initiate command is transmitted to the transmitter unit 102 (1110) when the receiver unit 104 is positioned within a close transmission range. Thereafter, in one aspect, the transmitter identification information is received (1120) for example, from the transmitter unit that received the sensor initiate command. Thereafter, a communication key (optionally encrypted) may be generated and transmitted (1130) to the transmitter unit.

In the manner described above, in one embodiment, a simplified pairing or synchronization between the transmitter unit 102 and the receiver unit 104 may be established using, for example, close proximity commands between the devices. As described above, in one aspect, upon pairing or synchronization, the transmitter unit 102 may be configured to periodically transmit analyte level information to the receiver unit for further processing.

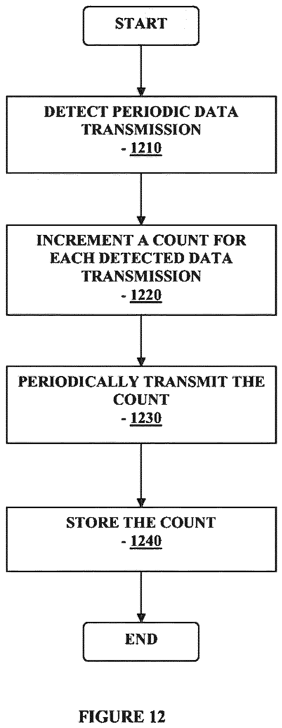

FIG. 12 is a flowchart illustrating the power supply determination in the data monitoring and management system of FIG. 1 in accordance with one embodiment of the present invention. That is, in one embodiment, using a counter, the receiver unit 104 may be configured to determine the power supply level of the transmitter unit 102 battery so as to determine a suitable time for replacement of the power supply or the transmitter unit 102 itself. Referring to FIG. 12, periodic data transmission is detected (1210), and a corresponding count in the counter is incremented for example, by one with each detected data transmission (1220). In particular, a Hobbs counter may be used in the rolling data configuration described above to provide a count that is associated with the transmitter unit data transmission occurrence.

Referring to FIG. 12, the updated or incremented count stored in the Hobbs counter is periodically transmitted in the data packet from the transmitter unit 102 to the receiver unit 104. Moreover, the incremented or updated count may be stored (1240) in a persistent nonvolatile memory unit of the transmitter unit 102. Accordingly, based on the number of data transmission occurrences, the battery power supply level may be estimated, and in turn, which may provide an indication as to when the battery (and thus the transmitter unit in the embodiment where the power supply is manufactured to be embedded within the transmitter unit housing) needs to be replaced.

Moreover, in one aspect, the incremented count in the Hobbs counter is stored in a persistent nonvolatile memory such that, the counter is not reset or otherwise restarted with each sensor unit replacement.

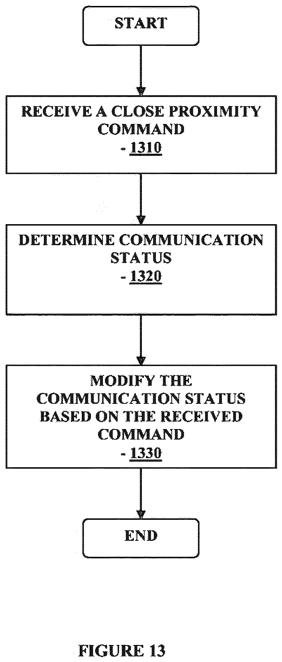

FIG. 13 is a flowchart illustrating close proximity command for RF communication control in the data monitoring and management system of FIG. 1 in accordance with one example. Referring to FIG. 13, a close proximity command associated with communication status, for example is received (1310). In one aspect, the command associated with the communication status may include, for example, a communication module turn on or turn off command for, for example, turning on or turning off the associated RF communication device of the transmitter unit 102. Referring to FIG. 13, the communication status is determined (1320), and thereafter, modified based on the received command (1330).

That is, in one aspect, using one or more close proximity commands, the receiver unit 104 may be configured to control the RF communication of the transmitter unit 102 to, for example, disable or turn off the RF communication functionality for a predetermined time period. This may be particularly useful when used in air travel or other locations such as hospital settings, where RF communication devices need to be disabled. In one aspect, the close proximity command may be used to either turn on or turn off the RF communication module of the transmitter unit 102, such that, when the receiver unit 104 is positioned in close proximity to the transmitter unit 102, and the RF command is transmitted, the transmitter unit 102 is configured, in one embodiment, to either turn off or turn on the RF communication capability of the transmitter unit 102.

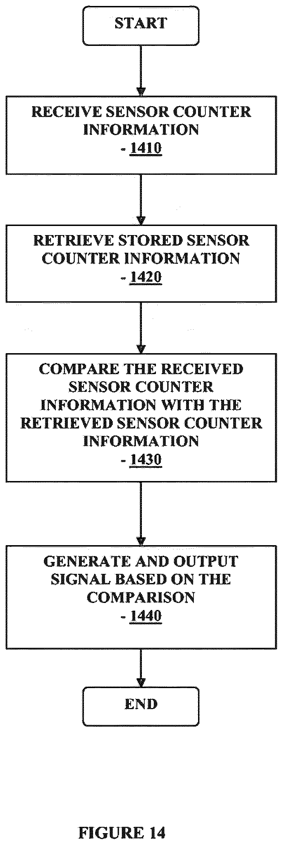

FIG. 14 is a flowchart illustrating analyte sensor identification routine in accordance with one example. Referring to FIG. 14, periodically, sensor counter information is received (1410), for example included as rolling data discussed above. The received sensor counter information may be stored in one or more storage units such as a memory unit. When the sensor counter information is received, a stored sensor counter information is retrieved (1420), and the retrieved sensor counter information is compared with the received sensor counter information (1430). Based on the comparison between the retrieved sensor counter information and the received sensor counter information, one or more signal is generated and output (1440).

That is, in one aspect, the sensor counter in the transmitter unit 102 may be configured to increment by one with each new sensor replacement. Thus, in one aspect, the sensor counter information may be associated with a particular sensor from which monitored analyte level information is generated and transmitted to the receiver unit 104. Accordingly, in one embodiment, based on the sensor counter information, the receiver unit 104 may be configured to ensure that the analyte related data is generated and received from the correct analyte sensor transmitted from the transmitter unit 102.

An analyte monitoring system in one aspect includes a data processing unit, and a control unit in wireless communication with the data processing unit, the control unit configured to transmit one or more predefined close proximity commands to the data processing unit, where the data processing unit is configured to perform one or more predefined functions in response to the received one or more predefined close proximity commands.

The data processing unit may include a close proximity receiver coupled to an antenna for receiving the one or more predefined close proximity commands from the control unit.

In one aspect, the data processing unit may be configured to transmit one or more signals related to a monitored analyte level in response to the received one or more predefined close proximity commands.

Further, the communication of the one or more predefined close proximity commands may be performed when the data processing unit and the control unit are within a predetermined distance from each other, where the predetermined distance may include a distance of less than 30.48 cm (one foot).

Also, there may be provided a memory unit, which may include an EEPROM or any other suitable type of nonvolatile or volatile memory, or combinations thereof.

In one embodiment, the control unit may include an application specific integrated circuit (ASIC) configuration.

The analyte sensor may be coupled to the data processing unit, where the data processing unit may receive one or more signals from the analyte sensor, and where the control unit and the data processing unit may be in wireless communication using RF communication protocol.

A method in accordance with one embodiment includes positioning a controller unit within a transmission range for close proximity communication, transmitting one or more predefined close proximity commands, and receiving a response packet in response to the transmitted one or more predefined close proximity commands.

The method may include generating a communication key associated with the response packet, where the communication key may be generated based on at least in part, a transmitter unit identification information, and further, where the transmitter unit identification information may be included in the response packet.

The transmission range in one embodiment may include a distance of less than 30.48 cm (one foot).

The method may also include storing one or more of the predefined close proximity commands or the response packet, where storing may include storing the commands or the response packet in a nonvolatile memory device.

The method may include receiving one or more signals from the analyte sensor, and further, where the transmitting and receiving include RF communication protocol.

An analyte sensor in one embodiment includes a substrate, a plurality of electrodes provided on the substrate, at least a portion of one of the plurality of electrodes positioned in fluid contact with an analyte of a user, and a conductive trace provided on the substrate and coupled to one of the plurality of electrodes.

The conductive trace may comprise carbon.

In one aspect, the conductive trace is connected to a ground terminal.

The plurality of electrodes may include one or more of a working electrode, a reference electrode and/or a counter electrode, and where the conductive trace may be connected to the counter electrode.

In one embodiment, the plurality of electrodes may be positioned in a stacked configuration.

The conductive trace in one aspect may be configured to establish electrical contact with a power supply to provide electrical signal to one or more of the plurality of electrodes.

Additionally, the conductive trace may be connected to a ground terminal, or alternatively, the conductive trace may be connected to a guard trace.

A system for powering a data processing device in accordance with another embodiment includes an analyte sensor, including a substrate, a plurality of electrodes provided on the substrate, at least a portion of one of the plurality of electrodes positioned in fluid contact with an analyte of a user, and a conductive trace provided on the substrate and coupled to one of the plurality of electrodes, a data processing device including a contact point for electrically connecting to one of the plurality of electrodes, the data processing device further including a power supply where when the contact point is in electrical connection with the one of the plurality of electrodes, the power supply is configured to transition the data processing device from a low power state to an active power state.

The power supply in one aspect includes a battery.

The one of the plurality of electrodes may include a counter electrode of the analyte sensor.

The conductive trace and the data processing device may be coupled to a ground terminal.

The sensor may include a guard trace disposed on the substrate, and further, where the data processing device may include a guard contact point for electrically coupling to the guard trace.

The data processing device may include a data communication unit to transmit one or more signals related to the monitored analyte level received from the analyte sensor, and further, where the data communication unit may include a close proximity receiver for receiving one or more close proximity commands.

The analyte sensor may include a glucose sensor.

Additionally, the analyte sensor may be transcutaneously positioned such that at least a portion of at least one of the plurality of electrodes is in fluid contact with an analyte of a user.

A method in accordance with still another embodiment includes detecting an electrical connection with an analyte sensor, and activating a data processing device to receive one or more analyte related signals from the analyte sensor.

The method may also include processing the one or more analyte related signals for wireless transmission.

An analyte monitoring device in one embodiment includes a data transmission section, a data receiving section, and a data processing section coupled to the data receiving section, where the data receiving section is configured to receive a predefined command, and further, where the data processing section is configured to control the data transmission section to transmit one or more data in response to the received predefined command.

The data receiving section may include a close proximity receiver.

The data transmission section may include an RF transmitter.

The device may include an antenna coupled to the data transmission section and the data receiving section for wireless data communication.

The data processing section may include a state machine.

The transmitted one or more data in one embodiment may include a device identification information.

In one aspect, the transmitted one or more data may include a response data packet, where the response data packet may include one or more of a communication mode information, a device status information, a device type information, a close proximity command information, or a sensor count information.

The device may include a memory unit for storing data.

A method in another embodiment includes receiving a command to initiate communication with an analyte sensor, retrieving an identification information, transmitting the retrieved identification information, and receiving a communication key associated with the transmitted identification information.

In one aspect, the received command may include a close proximity command.

Moreover, the retrieved identification information may be wirelessly transmitted over an RF communication link.

The identification information may include a data processing device identification information, where the data processing device identification information may include a transmitter identification information.

The communication key may include an 8 bit data associated with the identification information.

The method in another aspect includes transmitting a data packet including the communication key.

The method may also include storing the communication key.

In still another aspect, the analyte sensor includes a glucose sensor.

Moreover, the method includes receiving one or more signals related to a monitored analyte level, where the analyte level includes glucose level.

A method in one embodiment includes detecting a data transmission, incrementing a count associated with the detected data transmission, and storing the count.

The count may be incremented by one.

In a further aspect, the method may include associating a power supply level information with the stored count.

Moreover, the method may also include generating a signal associated with the stored count, and/or include outputting the generated signal, where outputting the generated signal may include one or more of visually displaying the generated signal, audibly outputting the generated signal, or vibratorily outputting the generated signal.

In yet another aspect, the method may include transmitting the count with the data transmission, where the count may be transmitted periodically with the data transmission.

In still another aspect, the method may include associating a power supply status with the count.

A data processing device in another embodiment may include a counter, a data communication unit, and a data processing section coupled to the data communication unit and the counter, the data processing section configured to increment a count stored in the counter based on data transmission by the data communication unit.

In one aspect, the counter may include a nonvolatile memory unit. The counter may include an EEPROM.

The data communication unit may include an RF transceiver.

The count stored in the counter may be incremented by one with each data transmission by the data communication unit.

The device may include a power supply coupled to the data processing unit, the data communication unit and the counter, where the count stored in the counter is not erased when the power supply is disabled or in low power state.

The data processing unit may be configured to estimate the power supply life based on the stored count in the counter.

The device in a further aspect may include an output section for outputting one or more signals associated with the count information, where the output section may include one or more of a display unit, an audible output section, or a vibratory output section.

An analyte monitoring system in one embodiment includes a data processing unit including a communication module, and a control unit in wireless communication with the data processing unit, the control unit configured to receive a predefined close proximity command associated with the wireless communication status of the data processing unit to the data processing unit, where in response to the received predefined close proximity command, the data processing unit changes the operational status of the communication module.

The predefined close proximity command may include one of an RF switch on command or an RF switch off command.

The data processing unit may modify the operational status of the communication module in response to the received predefined close proximity command, where the operational status may include one of an active status and an inactive status.

Additionally, when the communication module is in the inactive status, the data processing unit may be configured to not transmit any data to the control unit.

The control unit may include an output section to output information associated with the predefined close proximity command, where the output information may include one or more of a visual display output, an audible output, or a vibratory output.

In one aspect, the output information may include an icon, a visual display, a two or three dimensional image, video, or graphics, a audible output, an alarm, or combinations thereof.

A method in accordance with another embodiment includes determining a communication status, receiving a communication related close proximity command, and modifying the communication status based on the received command, where the communication status may include one of an RF communication module in active mode, or the RF communication module in inactive mode.

Also, modifying the communication status may include switching from the active mode to the inactive mode of the RF communication module, or from the inactive mode to the active mode of the RF communication module.

Additionally, modifying the communication status may include disabling wireless data transmission based on the received command.

The wireless data transmission may be disabled for a predetermined time period.

In another aspect, the method may include receiving a further communication related close proximity command, and further modifying the communication status based on the received further command, where further modifying may include activating the wireless data transmission based on the received further command.

The method in a further aspect includes transmitting data associated with monitored analyte level of a user, and further, also may include wirelessly transmitting the data associated with the monitored analyte level for further processing.

The method may include entering inactive operational mode based on the modified communication status.

In still another aspect, the method may include storing analyte related data received from an analyte sensor during the modified communication status period.

An analyte monitoring device in one embodiment includes a counter, and a data processing section coupled to the counter, the data processing section configured to increment a count stored in the counter based on a detection of an analyte sensor.

The counter may include a nonvolatile memory unit.

The data processing section may include a communication module to transmit data related to the count.

A control device for communicating with an analyte monitoring unit in one embodiment includes a data communication section configured to periodically receive data including a count information related to an analyte sensor associated with the received data, and a processing unit coupled to the data communication section to process the received data, the processing unit further configured to compare the count information in each data periodically received, and when it is determined that the count information of the periodically received data is different, the processing unit is configured to generate one or more signals related to the status of the analyte sensor.

In one aspect, the one or more signals generated may include a command to disable the data communication section.

The one or more signals generated may be provided to a user.

The device in a further aspect may include a display unit and where the one or more signals generated is displayed on the display unit.

In yet another aspect, the one or more signals output may include one or more of a visual indicator, an audible indicator or a vibratory indicator.

A method in accordance with yet another embodiment includes detecting an analyte sensor, updating a count associated with the detected analyte sensor, and transmitting the updated count.

The method may include storing the updated count.

Additionally, the method may include detecting a further analyte sensor, further updating the count associated with the detected further analyte sensor, and transmitting the further updated count.

A method in accordance with still another embodiment includes periodically receiving data including a count information related to an analyte sensor associated with the received data, and comparing the count information in each data periodically received, and when it is determined that the count information of the periodically received data is different, generating one or more signals related to the status of the analyte sensor.

The one or more signals generated may include a command to disable data communication.

The method may also include providing the one or more signals generated to a user, and also, include outputting the one or more signals generated.

In still a further aspect, the method may include storing the count information. The status of the analyte sensor may include a new analyte sensor detection. Additionally, the method may include prompting a user to replace the analyte sensor.

Various other modifications and alterations in the structure and method of operation of this invention will be apparent to those skilled in the art without departing from the scope of the invention. Although the invention has been described in connection with specific preferred embodiments, it should be understood that the invention as claimed should not be unduly limited to such specific embodiments. It is intended that the following claims define the scope of the present invention and that structures and methods within the scope of these claims be covered thereby.

## Claims

1. An analyte monitoring system, comprising:
a glucose sensor (101) for positioning in a transcutaneous manner such that at least a portion thereof is in fluid contact with an analyte of a user for monitoring a glucose level of the user; and
a data processing unit (102) coupled to the glucose sensor, and configured to generate glucose data from signals generated by the glucose sensor (101),
wherein the data processing unit (102) is configured to pair with a remote device (104) using an encrypted communication key, for establishing bi-directional communication between the data processing unit (102) and the remote device (104), the data processing unit (102) being configured to transmit (1020) identification information of the data processing unit (102) to the remote device (104) and the encrypted communication key being generated by the remote device (104) based on the identification information of the data processing unit (102), and the data processing unit (102) being further configured to receive (1030) the encrypted communication key from the remote device (104),
wherein, after the bi-directional communication is established and the data processing unit (102) is paired with the remote device (104), the data processing unit (102) is configured to transmit (1040) sensor related data with the encrypted communication key to the remote device (104) at predetermined time intervals,
wherein the data processing unit is configured to separate non-urgent data from urgent data, and to parse the non-urgent data into a number of data segments; and
wherein each transmission of sensor related data comprises all urgent data and a segment of non-urgent data.

2. The analyte monitoring system of claim 1, wherein the data processing unit (102) includes an application specific integrated circuit (ASIC).

3. The analyte monitoring system of either of claims 1 or 2, wherein the data processing unit (102) is in wireless communication with the remote device (104).

4. The analyte monitoring system of any preceding claim, wherein the data processing unit (102) includes a counter, and is configured to increment a count stored in the counter based on data transmission of the data processing unit (102).

5. The analyte monitoring system of any of claims 1 to 3, wherein the data processing unit (102) includes a counter, and is configured to increment a bit count stored in the counter, the incremented bit count corresponding to a remaining battery life of a battery coupled to the data processing unit (102).

6. The analyte monitoring system of any preceding claim, wherein the data processing unit (102) receives acknowledgement from the remote device (104) that the sensor related data has been received.

7. A method comprising:
transmitting (1020) identification information of a data processing unit (102) to a remote device (104);
generating an encrypted communication key at the remote device, the encrypted communication key being based on the identification information of the data processing unit (102),
receiving (1030) at the data processing unit the encrypted communication key from the remote device (104) to establish bi-directional communication between the data processing unit (102) and the remote device (104),
wherein the data processing unit (102) is coupled to a glucose sensor (101) that is configured for positioning in a transcutaneous manner such that at least a portion thereof is in fluid contact with an analyte of a user for monitoring a glucose level of the user, and
the method further comprising:
generating, at the data processing unit, glucose data from signals generated by the glucose sensor (101),
separating non-urgent data from urgent data, and parsing the non-urgent data into a number of data segments; and
after the bi-directional communication is established, transmitting (1040), from the data processing unit (102), sensor related data with the encrypted communication key to the remote device (104) at predetermined time intervals, wherein the step of transmitting sensor related data comprises transmitting all urgent data and a segment of non-urgent data.

8. The method of claim 7, wherein the data processing unit (102) includes an application specific integrated circuit (ASIC).

9. The method of either of claims 7 or 8, wherein the data processing unit (102) is in wireless communication with the remote device (104).

10. The method of any of claims 7 to 9, wherein the data processing unit (102) includes a counter, and the method further comprising incrementing a count stored in the counter based on data transmission of the data processing unit (102).

11. The method of any of claims 7 to 9, wherein the data processing unit (102) includes a counter, and the method further comprises incrementing a bit count stored in the counter, the incremented bit count corresponding to a remaining battery life of a battery coupled to the data processing unit (102).

12. The method of any of claims 7 to 11, further comprising receiving, at the data processing unit (102), acknowledgement from the remote device (104) that the sensor related data has been received.

13. The system of any of claims 1 to 6, or the method of any of claims 7 to 12, wherein the urgent data is glucose data from the glucose sensor and/or temperature data associated with the glucose sensor.

14. The system of any of claims 1 to 6 or 13, or the method of any of claims 7 to 13, wherein the non-urgent data is data associated with calibration.

## Patentansprüche

1. Ein Analytenüberwachungssystem, das Folgendes beinhaltet:
einen Glukosesensor (101) zur Positionierung in einer transkutanen Weise, sodass mindestens ein Anteil davon zur Überwachung des Glukosespiegels des Benutzers in Fluidkontakt mit einem Analyten eines Benutzers ist; und
eine Datenverarbeitungseinheit (102), die mit dem Glukosesensor gekoppelt ist und dazu ausgelegt ist, Glukosedaten aus durch den Glukosesensor (101) erzeugten Signalen zu erzeugen,
wobei die Datenverarbeitungseinheit (102) dazu ausgelegt ist, unter Verwendung eines verschlüsselten Kommunikationsschlüssels mit einer fernen Vorrichtung (104) gepaart zu werden, um eine bidirektionale Kommunikation zwischen der Datenverarbeitungseinheit (102) und der fernen Vorrichtung (104) herzustellen, wobei die Datenverarbeitungseinheit (102) zum Übermitteln (1020) von Kennungsinformationen der Datenverarbeitungseinheit (102) an die ferne Vorrichtung (104) ausgelegt ist und der verschlüsselte Kommunikationsschlüssel von der fernen Vorrichtung (104) basierend auf den Kennungsinformationen der Datenverarbeitungseinheit (102) erzeugt wird, und die Datenverarbeitungseinheit (102) ferner zum Empfangen (1030) des verschlüsselten Kommunikationsschlüssels von der fernen Vorrichtung (104) ausgelegt ist,
wobei nach dem Herstellen der bidirektionalen Kommunikation und dem Paaren der Datenverarbeitungseinheit (102) mit der fernen Vorrichtung (104) die Datenverarbeitungseinheit (102) zum Übermitteln (1040) von sensorbezogenen Daten mit dem verschlüsselten Kommunikationsschlüssel an die ferne Vorrichtung (104) in vorbestimmten Zeitintervallen ausgelegt ist,
wobei die Datenverarbeitungseinheit dazu ausgelegt ist, nicht dringende Daten von dringenden Daten zu trennen und die nicht dringenden Daten in eine Reihe von Datensegmenten zu parsen; und
wobei jede Übermittlung sensorbezogener Daten alle dringenden Daten und ein Segment von nicht dringenden Daten beinhaltet.

2. Analytenüberwachungssystem nach Anspruch 1, wobei die Datenverarbeitungseinheit (102) einen anwendungsspezifischen integrierten Schaltkreis (ASIC) beinhaltet.

3. Analytenüberwachungssystem nach einem der Ansprüche 1 oder 2, wobei die Datenverarbeitungseinheit (102) in drahtloser Kommunikation mit der fernen Vorrichtung (104) steht.

4. Analytenüberwachungssystem nach einem der vorhergehenden Ansprüche, wobei die Datenverarbeitungseinheit (102) einen Zähler umfasst und dazu ausgelegt ist, einen in dem Zähler gespeicherten Zählerstand basierend auf der Datenübermittlung der Datenverarbeitungseinheit (102) hochzuzählen.

5. Analytenüberwachungssystem nach einem der Ansprüche 1 bis 3, wobei die Datenverarbeitungseinheit (102) einen Zähler umfasst und dazu ausgelegt ist, einen in dem Zähler gespeicherten Bit-Zählerstand hochzuzählen, wobei der hochgezählte Bit-Zählerstand einer verbleibenden Batterielaufzeit einer mit der Datenverarbeitungseinheit (102) gekoppelten Batterie entspricht.

6. Analytenüberwachungssystem nach einem der vorhergehenden Ansprüche, wobei die Datenverarbeitungseinheit (102) eine Bestätigung von der fernen Vorrichtung (104), dass die sensorbezogenen Daten empfangen wurden, empfängt.

7. Ein Verfahren, das Folgendes beinhaltet:
Übermitteln (1020) von Kennungsinformationen einer Datenverarbeitungseinheit (102) an eine ferne Vorrichtung (104) ;
Erzeugen eines verschlüsselten Kommunikationsschlüssels an der fernen Vorrichtung, wobei der verschlüsselte Kommunikationsschlüssel auf den Kennungsinformationen der Datenverarbeitungseinheit (102) basiert,
Empfangen (1030), an der Datenverarbeitungseinheit, des verschlüsselten Kommunikationsschlüssels von der fernen Vorrichtung (104), um eine bidirektionale Kommunikation zwischen der Datenverarbeitungseinheit (102) und der fernen Vorrichtung (104) herzustellen,
wobei die Datenverarbeitungseinheit (102) mit einem Glukosesensor (101) gekoppelt ist, der zur Positionierung in einer transkutanen Weise ausgelegt ist, sodass mindestens ein Anteil davon zur Überwachung des Glukosespiegels des Benutzers in Fluidkontakt mit einem Analyten eines Benutzers steht, und
wobei das Verfahren ferner Folgendes beinhaltet:
Erzeugen, an der Datenverarbeitungseinheit, von Glukosedaten aus durch den Glukosesensor (101) erzeugten Signalen,
Trennen nicht dringender Daten von dringenden Daten und Parsen der nicht dringenden Daten in eine Reihe von Datensegmenten; und
nach dem Herstellen der bidirektionalen Kommunikation, Übermitteln (1040) sensorbezogener Daten von der Datenverarbeitungseinheit (102) mit dem verschlüsselten Kommunikationsschlüssel in vorbestimmten Zeitintervallen an die ferne Vorrichtung (104), wobei der Schritt des Übermittelns sensorbezogener Daten das Übermitteln aller dringenden Daten und eines Segments von nicht dringenden Daten beinhaltet.

8. Verfahren nach Anspruch 7, wobei die Datenverarbeitungseinheit (102) einen anwendungsspezifischen integrierten Schaltkreis (ASIC) beinhaltet.

9. Verfahren nach einem der Ansprüche 7 oder 8, wobei die Datenverarbeitungseinheit (102) in drahtloser Kommunikation mit der fernen Vorrichtung (104) steht.

10. Verfahren nach einem der Ansprüche 7 bis 9, wobei die Datenverarbeitungseinheit (102) einen Zähler umfasst und das Verfahren ferner das Hochzählen eines in dem Zähler gespeicherten Zählerstands basierend auf der Datenübermittlung der Datenverarbeitungseinheit (102) beinhaltet.

11. Verfahren nach einem der Ansprüche 7 bis 9, wobei die Datenverarbeitungseinheit (102) einen Zähler umfasst und das Verfahren ferner das Hochzählen eines in dem Zähler gespeicherten Bit-Zählerstands beinhaltet, wobei der hochgezählte Bit-Zählerstand einer verbleibenden Batterielaufzeit einer mit der Datenverarbeitungseinheit (102) gekoppelten Batterie entspricht.

12. Verfahren nach einem der Ansprüche 7 bis 11, das ferner das Empfangen, an der Datenverarbeitungseinheit (102), einer Bestätigung von der fernen Vorrichtung (104), dass die sensorbezogenen Daten empfangen wurden, beinhaltet.

13. System nach einem der Ansprüche 1 bis 6 oder Verfahren nach einem der Ansprüche 7 bis 12, wobei die dringenden Daten Glukosedaten von dem Glukosesensor und/oder mit dem Glukosesensor assoziierte Temperaturdaten sind.

14. System nach einem der Ansprüche 1 bis 6 oder 13 oder Verfahren nach einem der Ansprüche 7 bis 13, wobei die nicht dringenden Daten mit der Kalibrierung assoziierte Daten sind.

## Revendications

1. Système de surveillance d'analyte, comprenant :
un capteur de glucose (101) destiné à un positionnement transcutané de telle sorte qu'au moins une partie de celui-ci soit en contact fluidique avec un analyte d'un utilisateur pour la surveillance d'un taux de glucose de l'utilisateur ; et
une unité de traitement de données (102) couplée au capteur de glucose, et configurée pour générer des données sur le glucose à partir de signaux générés par le capteur de glucose (101),
dans lequel l'unité de traitement de données (102) est configurée pour être appariée avec un dispositif distant (104) à l'aide d'une clé de communication cryptée, pour établir une communication bidirectionnelle entre l'unité de traitement de données (102) et le dispositif distant (104), l'unité de traitement de données (102) étant configurée pour transmettre (1020) des informations d'identification de l'unité de traitement de données (102) au dispositif distant (104) et la clé de communication cryptée étant générée par le dispositif distant (104) sur la base des informations d'identification de l'unité de traitement de données (102), et l'unité de traitement de données (102) étant configurée en outre pour recevoir (1030) la clé de communication cryptée à partir du dispositif distant (104),
dans lequel, après que la communication bidirectionnelle a été établie et que l'unité de traitement de données (102) a été appariée avec le dispositif distant (104), l'unité de traitement de données (102) est configurée pour transmettre (1040) les données relatives au capteur avec la clé de communication cryptée au dispositif distant (104) à des intervalles de temps prédéterminés,
dans lequel l'unité de traitement de données est configurée pour séparer les données non urgentes des données urgentes, et pour redistribuer les données non urgentes en plusieurs segments de données ; et
dans lequel chaque transmission de données relatives au capteur comprend toutes les données urgentes et un segment de données non urgentes.

2. Système de surveillance d'analyte selon la revendication 1, dans lequel l'unité de traitement de données (102) comprend un circuit intégré spécifique à l'application (ASIC).

3. Système de surveillance d'analyte selon l'une ou l'autre des revendications 1 ou 2, dans lequel l'unité de traitement de données (102) est en communication sans fil avec le dispositif distant (104).

4. Système de surveillance d'analyte selon l'une quelconque des revendications précédentes, dans lequel l'unité de traitement de données (102) comprend un compteur, et est configurée pour incrémenter un compte enregistré dans le compteur sur la base de la transmission de données de l'unité de traitement de données (102).

5. Système de surveillance d'analyte selon l'une quelconque des revendications 1 à 3, dans lequel l'unité de traitement de données (102) comprend un compteur, et est configurée pour incrémenter un nombre de bits enregistré dans le compteur, le nombre de bits incrémenté correspondant à une durée de vie restante d'une pile couplée à l'unité de traitement de données (102).

6. Système de surveillance d'analyte selon l'une quelconque des revendications précédentes, dans lequel l'unité de traitement de données (102) reçoit un accusé de réception à partir du dispositif distant (104) indiquant que les données relatives au capteur ont été reçues.

7. Procédé comprenant :
la transmission (1020) d'informations d'identification d'une unité de traitement de données (102) à un dispositif distant (104) ;
la génération d'une clé de communication cryptée au niveau du dispositif distant, la clé de communication cryptée étant basée sur les informations d'identification de l'unité de traitement de données (102),
la réception (1030) au niveau de l'unité de traitement de données de la clé de communication cryptée à partir du dispositif distant (104) pour établir une communication bidirectionnelle entre l'unité de traitement de données (102) et le dispositif distant (104),
dans lequel l'unité de traitement de données (102) est couplée à un capteur de glucose (101) qui est configuré pour un positionnement transcutané de telle sorte qu'au moins une partie de celui-ci soit en contact fluidique avec un analyte d'un utilisateur pour la surveillance d'un taux de glucose de l'utilisateur, et
le procédé comprenant en outre :
la génération, au niveau de l'unité de traitement de données, de données sur le glucose à partir de signaux générés par le capteur de glucose (101),
la séparation des données non urgentes des données urgentes, et la redistribution des données non urgentes en plusieurs segments de données ; et
après que la communication bidirectionnelle a été établie, la transmission (1040), à partir de l'unité de traitement de données (102), de données relatives au capteur avec la clé de communication cryptée au dispositif distant (104) à des intervalles de temps prédéterminés, l'étape de transmission de données relatives au capteur comprenant la transmission de toutes les données urgentes et d'un segment de données non urgentes.

8. Procédé selon la revendication 7, dans lequel l'unité de traitement de données (102) comprend un circuit intégré spécifique à l'application (ASIC).

9. Procédé selon l'une ou l'autre des revendications 7 ou 8, dans lequel l'unité de traitement de données (102) est en communication sans fil avec le dispositif distant (104) .

10. Procédé selon l'une quelconque des revendications 7 à 9, dans lequel l'unité de traitement de données (102) comprend un compteur, et le procédé comprenant en outre l'incrémentation d'un compte enregistré dans le compteur sur la base de la transmission de données de l'unité de traitement de données (102).

11. Procédé selon l'une quelconque des revendications 7 à 9, dans lequel l'unité de traitement de données (102) comprend un compteur, et le procédé comprenant en outre l'incrémentation d'un nombre de bits enregistré dans le compteur, le nombre de bits incrémenté correspondant à une durée de vie restante d'une pile couplée à l'unité de traitement de données (102).

12. Procédé selon l'une quelconque des revendications 7 à 11, comprenant en outre la réception, au niveau de l'unité de traitement de données (102), d'un accusé de réception à partir du dispositif distant (104) indiquant que les données relatives au capteur ont été reçues.

13. Système selon l'une quelconque des revendications 1 à 6, ou procédé selon l'une quelconque des revendications 7 à 12, dans lequel les données urgentes sont des données sur le glucose provenant du capteur de glucose et/ou des données de température associées au capteur de glucose.

14. Système selon l'une quelconque des revendications 1 à 6 ou 13, ou procédé selon l'une quelconque des revendications 7 à 13, dans lequel les données non urgentes sont des données associées à un étalonnage.
